# EUROPEAN PATENT APPLICATION

(11) **EP 1 857 558 A1**
(43) Date of publication of application: **21.11.2007**
(21) Application number: 05806777.8
(22) Date of filing: 15.11.2005
(51) Int. Cl.: C12P 21/02, C12N 15/09

(54) **CELL-FREE PROTEIN SYNTHESIS SYSTEM FOR SYNTHESIZING GLYCOPROTEIN**

(30) Priority: 17.11.2004 JP 2004333251
(71) Applicant: Riken, Wako-shi, Saitama 351-0198 (JP)
(72) Inventor: IMATAKA, Hiroaki, c/o RIKEN Yokohama Institute, Yokohama-shi, Kanagawa 2300045 (JP); MIKAMI, Satoshi, c/o RIKEN Yokohama Institute, Yokohama-shi,Kanagawa 2300045 (JP); YOKOYAMA, Shigeyuki, c/o RIKEN Yokohama Institute, Yokohama-shi, Kanagawa 2300045 (JP)
(74) Representative: Arends, William Gerrit
(86) International application number: PCT/JP2005/020961
(87) International publication number: WO 2006/054557

(57) **Abstract**

Provided is a process for producing a protein with post-translational modification in a cell-free protein synthesis system having a higher protein synthetic activity and glycosylation capability. The process of the present invention comprises preparing a cell extract from cultured cells of an immortalized mammalian cell line that has enhanced protein secreting activity, and adding an mRNA encoding a glycoprotein to the cell extract.

SEQUENCE LISTING

<110> RIKEN
IMATAKA, Hiroaki
MIKAMI, Satoshi
YOKOYAMA, Shigeyuki

<120> Cell-free protein synthesis system for synthesizing glycoprotein

<130> 20406

<150> JP2004-333251
<151> 2004-11-17

<160> 6

<170> PatentIn version 3.1

<210> 1
<211> 38
<212> DNA
<213> Artificial Sequence

<220>
<223> primer

<400> 1
ccgggagctc cagagtgaag gagaagtatc agcacttg 38

<210> 2
<211> 40
<212> DNA
<213> Artificial Sequence

<220>
<223> primer

<400> 2
tctcgatatc tcatcttttt tctctctgca ccactcttct 40

<210> 3
<211> 37
<212> DNA
<213> Artificial Sequence

<220>
<223>  primer

<400>  3
ccggccgagc tcggcaacag ggaatctgcc cggttgc                                37

<210>  4
<211>  40
<212>  DNA
<213>  Artificial Sequence

<220>
<223>  primer

<400>  4
tctcgatatc tcaggcctca gcctgggcta tcagcagcat                             40

<210>  5
<211>  37
<212>  DNA
<213>  Artificial Sequence

<220>
<223>  primer

<400>  5
ccggccgagc tcggtgggga actgggctaa ggtcttg                                37

<210>  6
<211>  40
<212>  DNA
<213>  Artificial Sequence

<220>
<223>  primer

<400>  6
tctcgatatc tcaggcctca gcctgggcta tcagcagcat                             40

## Description

### TECHNICAL FIELD

The present invention relates to a process for synthesizing a protein in cell-free system, in particular, a system for synthesizing glycoprotein using an extract derived from mammalian cells.

### BACKGROUND ART

More than one third of mammalian proteins are predicted to have an addition of carbohydrate chains (glycans). A number of studies show the carbohydrate chains added to proteins closely relate to development, differentiation, growth and malignant transformation. Thus, addition of carbohydrate chains is essential for exhibiting activities of many proteins used for medical products and the like. It is also necessary to prepare glycoproteins for biochemical analysis thereof by recombinant DNA technologies. There is a method of expressing glycoproteins in living cells and purifying them, however, it is more convenient to synthesize glycoproteins in a cell-free system. Synthesis of glycoproteins in cell-free system is generally carried out by using rabbit reticulocyte lysates combined with microsomes from dog pancreas (an intracellular organelle for glycosylation; refer to, for example, Non-patent Document 1). The canine pancreatic microsomes are commercially available, but they are expensive and activities thereof have large lot-to-lot variances. In addition, most of ordinary researchers are difficult to obtain the canine pancreas to prepare the microsome fraction by themselves. Therefore, a cell-free method of producing proteins with complete post-translational modifications has been reported, in that machineries of post-translational modification including glycosylation (endoplasmic reticulum, Golgi apparatus, cell membrane etc.) are prepared from tissue cells or cultured cells such as Chinese hamster ovary (CHO) cells, and added to the rabbit reticulocyte lysate to synthesize glycoproteins (see Patent Document 1).

Meanwhile, it is very important to produce glycoproteins simply and stably for the research and drug development using glycoproteins. In particular, this is critical point in the research for preventing infectious diseases. Human hepatitis C virus (HCV) and human immunodeficiency virus (HIV) have envelopes consisting of glycoproteins, and it is thought that carbohydrate chains of the envelope are important for infections. Since both HCV and HIV are infectious to only human cells, it is preferable to synthesize these envelope proteins in human cells for their biological analysis.

[Patent Document 1]
   Japanese Patent Kokai publication No. JP-A-2002-238595
[Non-patent Document 1]
   Walter, P. and Blobel, G. (1983) Method. Enzymol. , Vol.96, pp.84

### SUMMARY OF THE INVENTION

### PROBLEMS TO BE SOLVED BY THE INVENTION

It takes a lot of time and work to prepare the machineries of post-translational modification derived from canine pancreatic microsomes or cultured cells used in the conventional art. The cell-free protein synthesis system consisting of these machineries combined with extract components derived from rabbits is a heterogeneous system, and some components might be defective for post-translational modification. It is an object of the present invention to provide a process of producing a protein in a cell-free protein synthesis system using an extract of mammalian cells, which process has a higher protein synthetic activity and glycosylation capability.

### MEANS FOR SOLVING THE PROBLEM

The present inventors made every effort to solve the problems above, and in consequence, have found that a cell extract of hybridoma used for producing a monoclonal antibody, was capable of synthesizing a glycoprotein with high efficiency. On the basis of this finding, the present invention has been completed.

According to a first aspect of the present invention, there is provided a process for producing a protein with a post-translational modification in a cell-free protein synthesis system comprising preparing a cell extract from cultured cells of an immortalized mammalian cell line that has enhanced protein secreting activity, and adding an mRNA encoding a glycoprotein to the cell extract. The cultured cells are preferably suspension cells, and are more preferably hybridoma cells that produce a monoclonal antibody. The mRNA encoding a glycoprotein can be a virus-derived RNA.

In another aspect of the present invention, there is provided a composition for use in a synthesis of protein in cell-free system, comprising a cell extract from cultured cells of an immortalized mammalian cell line that has enhanced protein secreting activity. It is preferable that the composition further comprises an amino acid, a buffer solution, a salt, a nucleotidetriphosphate, and an energy substance.

In a preferable example of the present invention, the cultured cells are hybridoma cells that produce a monoclonal antibody, and most preferably derived from human.

### ADVANTAGE OF THE INVENTION

According to the present invention, proteins with a post-translational modification, such as glycoproteins can be produced by means of using a cell extract from cultured cells that have an enhanced protein secreting activity. The preparation of the extract is also easy by using cultured cells of an immortalized mammalian cell line. In particular, antibody-producing hybridomas are suspension cells, which are easy for use in a large scale cultivation, and most of them can be grown easily in a serum-free medium. Further, hybridomas derived from human cells make it possible to easily prepare glycoproteins that have carbohydrate chains of human type. Such a method of synthesizing glycoproteins is useful for the study of structures and functions thereof in human cells.

### BRIEF DESCRIPTION OF THE DRAWINGS

Fig. 1 is a schematic representation of plasmid DNA structures for synthesizing each of the mRNAs used in Examples of this application.
Fig. 2 shows a result of SDS-PAGE analysis of the HIV envelope proteins synthesized by cell-free translation reactions derived from human.
Fig. 3 shows a result of SDS-PAGE analysis of the HCV envelope proteins synthesized by cell-free translation reactions derived from human.

### BEST MODE FOR CARRYING OUT THE INVENTION

### [Cell-free protein synthesis system]

As used herein, a cell-free protein synthesis system is a system to synthesize a target protein by preparing an extract including protein factors necessary for translation of proteins, and reconstituting the reaction *in vitro.* The extracts derived from a variety of organisms can be used for constituting the cell-free system, for example, those of eukaryotic and prokaryotic cells with high protein synthetic activities, such as bacteria including *E.coli,* thermophilic bacteria and the like, wheat germ, rabbit reticulocyte, mouse L cell, Ehrlich's ascitic cancer cell, HeLa cell, CHO cell and budding yeast and the like (see, Clemens, M.J., Transcription and Translation - A Practical Approach, (1984), pp. 231-270; Henes, B.D. et al. eds., IRL Press, Oxford).

In a method of the present invention, the cell extract for cell-free protein synthesis is prepared from cultured cells of an immortalized mammalian cell line that has enhanced protein secreting activity. Mammalian cells are not limited to specifically, but such as mouse L cell, Ehrlich's ascitic cancer cell, HeLa cell, and CHO cell can be used. Among these cells, those that have a high protein secreting activity, or artificially modified cells as such are preferable. The preferable examples of such cells are hybridoma cells that produce monoclonal antibody as described below. Since the cell secretes a glycoprotein, antibody molecule, it is expected that membranous organs such as endoplasmic reticulum and Golgi apparatus in the cell have been grown as machineries for addition of carbohydrate chain. Hybridoma cells are easy for culture as suspension cells, and most of them can be grown efficiently in a serum-free medium.

### [Preparation of hybr.idoma cells producing monoclonal antibody]

Hybridoma cell lines may be prepared conventionally, such as by the method of Koehler and Milstein "Nature, Vol. 256, pp. 495-497(1975)", and "European Journal of Immunol., Vol. 6, pp.511-519 (1976)". In a concrete method, an antigen used as an immunogen is not particularly limited, but any heterologous proteins or peptides for the immunized animal can be used. It is difficult to produce an antibody by immunizing low molecular weight compounds *per se,* thus, they are usually coupled with a carrier protein such as KLH or albumin. As for immunized animals, any mammals can be used without limitation, but such mammals as mouse, rat and hamster can be used. In case of mouse, for example, stimulated lymphocytes that have been immunized by sufficient amount of antigen are prepared as follows. Approximately 25 µg per mouse of antigen are primed intraperitoneally about three times at intervals of two weeks, and then further 25 µg are immunized intravenously. After several days of final immunization, spleen cells are removed for fusion.

From the individual mammals immunized as described above, spleen is removed aseptically, and a single cell suspension is prepared from the spleen. The spleen cells (antibody producing cells) are fused with appropriate myeloma cells using a suitable accelerator of cell fusion. As a type of myeloma cells, those derived from the same mammalian species with the immunized animal are preferable, but it is also possible to fuse spleen cells such as rat and hamster with myeloma cells of mouse. Suitable accelerator of cell fusion is, for example, Polyethyleneglycol having a average molecular weight between 1000 and 4000 (daltons).

The mixture of non-fused spleen cells, non-fused myeloma cells and fused cells are diluted with a selection medium that does not support the non-fused myeloma cells, and cultured for only a sufficient time to kill the non-fused cells (about one hour). The culture media such as HAT medium are used, which do not support the non-fused myeloma cells of a drug resistance such as 8-azaguanine resistance. The non-fused myeloma cells cannot be grown in this medium, and the non-fused spleen cells also die after a certain period of time, for example one week, since they are non-tumor cells. Thus remaining cells can be alive in the selection medium since they have both of neoplastic character of parent myeloma cells and antibody producing character of spleen cells. In addition, they have further an enhanced activity of protein secretion. It is further possible to select a high-antibody secreting cell line by cloning using an appropriate method such as a limiting dilution method.

The first attempt to obtain human-human hybridomas by means of hybridizing a parent cell line having only human chromosomes and a human antibody producing cell line were reported on 1980 by Olsson and Kaplan and Croce et al. (L. Olsson and H.S. Kaplan, Proc. Natl. Acad. Sci. USA, 77, 5429 (1980); and C.M. Croce et al., Nature, 288, 488 (1980)), and then preparations of a variety of human-human hybridomas have been reported. For example, there are a number of hybrodomas prepared by fusion of human B lymphocytes derived from a living subject and myeloma cells, or fusion of human B lymphocyte cell line established by EB virus etc. and myeloma cells. The myeloma cell used herein includes a human myeloma cell represented by U266, a mouce myeloma cell represented by P3U1. It is also possible to use other human myeloma cells, or human lymphoblastic cell lines such as Namalwa cell, or any myeloma cell derived from other animal species.

### [Preparation of composition for synthesizing a protein in cell-free system]

The established hybridoma cells can be easily cultured by any culture apparatus known to a person skilled in the art. Next, the cultured cells are mechanically disrupted using a known method such as a homogenizer. The obtained extract is treated with a Micrococcal nuclease and CaCl₂ to degrade endogenous mRNAs and reduce the background translation to minimum level. Then, EGTA is added to the extract for chelating CaCl₂, thereby inactivating the nuclease.

In one example of the present invention, the cultured mammalian cells are disrupted by exposing to a compressed inert gas (e.g. nitrogen gas) to pressurize and then reducing the pressure. For example, Mini-Bomb Cell Disruption Chamber is commercially available by KONTES for this purpose. In this method, the cells are cooled by expanding gas without temperature increase which is observed in the methods of mechanical disruption and sonication and the like. Thus, it is possible to adjust the degree of cellular destruction by altering the pressure or equilibrium time. For example, when the cells are treated under medium pressure for a short period of time, it is possible to disrupt cell membrane only without disrupting intracellular organelles. It has been reported that a high synthesis activity of glycoprotein was obtained by preparing an extract of insect cells with this apparatus (see Tarui, H. et al., Appl. Microbiol. Biotechnol. Vol. 55, pp.446-453, 2001).

The extracts comprise several components necessary for protein synthesis such as tRNAs, rRNAs, amino acids, as well as initiation factors, elongation factors and termination factors. The translation reaction of mRNA can be further optimized by addition of an energy regenerating system comprising phosphocreatine kinase and phosphocreatine. A mixture of amino acids may further be added. Potassium acetate and magnesium acetate may also be added to adjust their concentrations suitable for translation reaction.

The reaction mixture or amino acid mixture for use in a cell-free protein synthesis can be dispensed easy for use in aliquots in separate or previously mixed form, and distributed as a product. These products can be stored at frozen state or dried state, and put on the market as a kit in suitable containers for storage and for shipment. The kit can be accompanied by an instruction manual, a positive control DNA, a vector DNA and the like.

### [Synthesis of glycoproteins]

Template mRNAs used for cell-free protein synthesis are those encoding target glycoproteins to be expressed without any limitation, and can be synthesized by, for example, isolating the mRNA directly from the cell, or cloning a DNA encoding the glycoprotein in a vector with an RNA polymerase promoter, and carrying out in vitro transcription reaction. A method of in vitro transcription is known, in which a cloned DNA is inserted downstream to a phage polymerase promoter (Krieq, P. and Melton, D., Nucl. Acids Res. Vol. 12, p.7057, 1984). According to this method, a target gene to be expressed is cloned in a vector that has any one of promoters of SP6, T7 and T3 polymerases. Then, after linearizing the vector at its 3' end of the cloned gene with a restriction enzyme, *in vitro* transcription reaction is performed to synthesize mRNA. A number of vectors having any one of promoters of SP6, T7 and T3 polymerases are commercially available, and can be obtained easily. Thus synthesized template mRNA may have a cap structure at its 5' end, or not. In case of adding a cap at 5' end of the mRNA, a cap analog may be used as a substrate to synthesize a capping mRNA by the enzymatic reaction with RNA polymerase. Glycoproteins encoded by the template DNAs are those for medicament and research, and include but not limited to, for example, growth hormones, granulocyte colony-stimulating factor, interleukins, interferons, thrombopoietin, erythropoietin, tissue-plasminogen activator, humanized nomoclonal antibody and the like.

In one example of the present invention, mRNAs derived from viruses can be used for the template. For example, the major envelope glycoprotein (gp120) encoded by mRNA of HIV consists of a polypeptide of about 60 kDa and 21 N-glycosylation sites (Asn-Xaa-Ser/Thr) which increase the total molecular weight to 100-120 kDa. Thus, gp120 synthesized in the above cell-free system can be used for detecting the presence or absence of the glycosylation by measuring a change of the molecular weight after treatment with Endoglycosidase H.

In another example, an internal ribosome entry site (IRES) of Picornavirus may be added at 5' end of the mRNA. It is known that the translation reaction of mRNAs of Picornavirus is initiated by using the internal ribosome entry site (IRES). The structure of IRES of Picornavirus is roughly classified into two types. One is those of Enterovirus such as Poliovirus, and Rhinovirus, and another is those of Cardiovirus such as Encephalomyocarditis virus (EMCV) and Aphthovirus such as Foot-and-mouth disease virus. For example, translation of EMCV RNA is initiated from the IRES at the 5' untranslated region thereof, and a large polyprotein is synthesized. The polyprotein is cleaved by virus-derived protease 3C^{pro} or 3ABC^{pro}, and processed to mature virus proteins. The full-length sequence of EMCV genome has been registered in, for example, GenBankAccessionNos. NC_001497, X87335 and the like. The IRES exists in the 5'-untranslated region of EMCV RNA genome, and the sequence can be cloned by, for example, amplifying a region from position 281 to position 848 of EMCV genome with PCR.

### EXAMPLES

The present invention is explained in more details by reference to the following examples using a human hybridoma cell line HF10B4, however these examples are typical concrete cases and do not intend to limit the scope of the present invention.

### [Cell culture]

The culture of HF10B4 cells (RIKEN BioResource Center, Cell No.RCB0708) using a Petri dish was carried out in a CO₂ incubator (5% CO₂ concentration, at 37°C), in E-RDF medium (KYOKUTO) supplemented with 10% heat-inactivated fatal calf serum (ICN Biomedicals, Inc.) and L-glutamate (2mM).

The spinner culture in one-litter scale was performed using a spinner flask equipped with a cell culture controller system (Cell Master Model 1700, WAKENYAKU Co., Ltd.). The control values of temperature, pH, dissolved oxygen concentration, and stirring speed were 37°C, 7.0, 6.7ppm, and 20rpm, respectively.

The culture of HeLa S3 cells using a Petri dish was also carried out in a CO₂ incubator (5% CO₂ concentration, at 37°C), in Eagle's MEM medium (SIGMA) supplemented with 10% heat-inactivated fetal calf serum (ICN Biomedicals, Inc.), L-glutamate (2mM), 1 unit/ml penicillin, and 0.1mg/ml streptomycin. The spinner culture of HeLa S3 cells was performed at the same conditions except that the pH and stirring speed were set at 7.2 and 50rpm respectively.

### [Preparation of cell extract]

When the cell density reached 1.0 - 1.5×10⁶/ml, the HF10B4 cells in the culture broth were harvested by centrifugation at 360×g and 4°C, for 10 minutes. The culture medium was removed by aspiration, and the cells obtained from one liter culture were moderately suspended in 80 ml of buffer (35mM HEPES-KOH (pH 7.5 at 25°C), 140mM NaCl, 11mM glucose) , and then transferred to two 50ml tubes to centrifuge at 360×g and 4°C, for 5 minutes. After repeating the procedure three times, 1.5 volumes of the extract buffer (20mM HEPES-KOH (pH 7.5 at 25°C), 45mM potassium acetate, 45mM KC1, 1.8mM magnesium acetate) relative to the cell-volume was added, and the cells suspended in a density of approximately 2.5×10⁸/ml were entrapped in a Mini-Bomb Cell Disruption Chamber (KONTES). The cells were disrupted by nitrogen pressure of 1.0Mpa in a standing state on ice. After 30 minutes, the cell homogenate was recovered in a flow rate of about 3 to 10 drops/sec by gradually opening the outlet port. Thus obtained cell homogenate was centrifuged at 700xg, for 5 minutes at 4°C twice to remove the presipitate, and the residue (2 - 2.5ml) was applied to PD-10 column (Amersham) equilibrated with the extraction buffer previously. The sample was loaded on the column by spontaneous fall, and then an additional extract buffer was charged on the column. When the color of dropping solution from the column is changed from transparent to opal, approximate 1.7 to 2.0ml solution was harvested as a cell extract. The obtained extract was divided into equal amount of aliquots, quickly frozen in liquid nitrogen, and then stored at -80°C. HeLa S3 cell extract was also prepared by the same method of HF10B4 cells.

### [Preparation of plasmid and mRNA]

To synthesize mRNAs encoding the envelope proteins of each of HIV and HCV, plasmids pSP72-EMCV-HIVgp120-poly-A, pSP72-EMCV-HCV-E1-E2-poly-A, and pSP72-EMCV-HCV-E2-poly-A were prepared. The methods for constructing the plasmids and methods for synthesizing mRNAs encoded thereby are as follows:

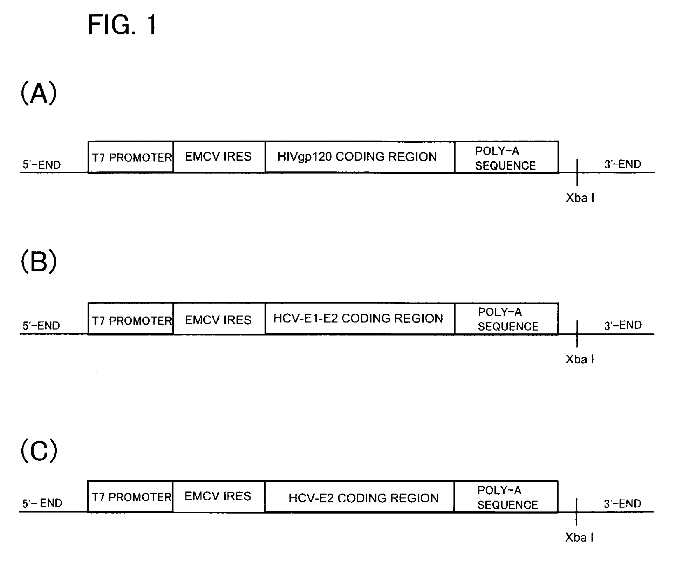

An expression vector for gp120 of HIV, pSP72-EMCV-HIVgp120-poly-A was prepared firstly by amplifying a DNA fragment encoding gp120 corresponding to an amino acid sequence from position 2 to position 509 of HIV-gp160. A PCR was carried out with a plasmid pNM3rM (Kuwata et al., (1995) J. Gen. Virol. 76:2181-2191) as a template using a primer : 5'-CCGGGAGCTCCAGAGTGAAGGAGAAGTATCAGCACTTG-3' (SEQ ID NO.1) and a primer:
5'-TCTCGATATCTCATCTTTTTTCTCTCTGCACCACTCTTCT-3' (SEQ ID NO.2)
The amplified DNA fragment was used to replace the luciferase coding region of a plasmid pSP72-EMCV-Luc-poly-A (Svitkin, et al. (2001) RNA 7:1743-1752). Fig.1(A) is a schematic diagram of the structure of plasmid pSP72-EMCV-HIVgp120-poly-A prepared as described above.

Synthesis of EMCV-HIVgp120-poly-A mRNA was carried out according to the method of RiboMAX Large Scale RNA Production Systems (Promega) with T7 RNA Polymerase. The template DNA was prepared by cleaving the plasmid pSP72- EMCV-HIVgp120-poly-A at its 3' side of polyA sequence with a restriction enzyme XbaI.

An expression vector for E1-E2 of HCV, pSP72-EMCV-HCV-E1-E2-poly-A was prepared by amplifying a DNA fragment encoding E1-E2 corresponding to an amino acid sequence from position 165 to position 746 of full-length polypeptide of HCV. The PCR was carried out with a plasmid pCMV-980 (Marusawa et al. (1999) J. Virol. 73: 4713-4720) as a template, and using the primers :
5'-CCGGCCGAGCTCGGCAACAGGGAATCTGCCCGGTTGC-3' (SEQ ID NO.3) and
5'-TCTCGATATCTCAGGCCTCAGCCTGGGCTATCAGCAGCAT-3' (SEQ ID No.4).
Next, the amplified DNA fragment was used to replace the coding region of luciferase of a plasmid pSP72-EMCV-Luc-poly-A as described above.

An expression vector for E2 of HCV, pSP72-EMCV-HCV-E2-poly-A was prepared in the same way by amplifying a DNA fragment encoding E2 corresponding to an amino acid sequence from position 365 to position 746 of full-length polypeptide of HCV, using the following two primers :
5'-CCGGCCGAGCTCGGTGGGGAACTGGGCTAAGGTCTTG-3' (SEQ ID NO.5) and
5'-TCTCGATATCTCAGGCCTCAGCCTGGGCTATCAGCAGCAT-3' (SEQ ID NO.6) .
The amplified DNA fragment was used to replace the coding region of luciferase of a plasmid pSP72-EMCV-Luc-poly-A. Fig.1 (B) and Fig.1 (C) are schematic diagrams of the structure of plasmids pSP72-EMCV-HCV-E1-E2-poly-A and pSP72-EMCV-HCV-E2-poly-A, respectively prepared as described above.

Synthesis of EMCV-HCV-E1-E2-poly-A mRNA and EMCV-HCV-E2-poly-A mRNA were carried out according to the method of RiboMAX Large Scale RNA Production Systems (Promega) with T7 RNA Polymerase. The template DNAs were prepared by cleaving the plasmids pSP72-EMCV-HCV-E1-E2-poly-A and pSP72-EMCV-HCV-E2-poly-A at their 3' sides of polyA sequence with a restriction enzyme XbaI. The concentration of each of the synthesized mRNAs was determined by measuring absorbance at a wavelength 260nm, and stored at -80°C.

### [Cell-free protein synthesis]

Prior to the translation reaction of each of mRNAs encoding the envelope proteins derived from viruses, endogenous RNAs in the extract were decomposed and removed according to the following method. To 100 µl of the extract (20 - 26 mg/ml of protein concentration), 1µl of nuclease S7 (7500 units/ml) and 1µl of 100mM CaCl₂ were added. After incubation at 20°C for 5 min, the reaction was stopped by adding 8 µl of 30mM EGTA. Then, to 110µl of the extract, 16.4µl of solution of energy mixture (184mM HEPES-KOH (pH7.5), 13.2mM ATP, 1.32mM GTP, 198mM creatine phosphate, 3.3mM spermidine, 13.2mM magnesium acetate), 4.92 µl of calf liver t-RNA (3.3 mg/ml), 1.8 µl of creatine kinase (6.0 mg/ml),9 µl of 2.0M KCl, 1.8 µl of 50 mM magnesium acetate and 16.2 µl of H₂O were added to prepare a reaction mixture. The reaction mixture was further charged into a dialysis chamber (Cut-off M.W. 50 kDa, recycle cellulose), and dialyzed at 27°C for 1.5-3 hours against 4.8 ml of external dialysate containing 490 µl of energy-mixture, 3.0 ml of the extract buffer, 270 µl of 2.0 M KCl, 27 µl of 100 mM magnesium acetate, 16.2 µl of 400 mM EGTA.

The translation reaction was performed in 8 µl of the reaction mixture transferred in an Eppendorf tube, supplemented in turn and mixed with 0.5 µl of 2.5 mCi/ml ³⁵S-methionine, and 0.5 µl of each of 0.6 µg/µl mRNA solution (total 9 µl), and then incubated for one hour at 27°C. Final concentration of each of the compositions included in the reaction mixture is as follows: 11-14mg proteins from the extract/ml, 27mM HEPES-KOH (pH7.5), 15 µM of each of 19 amino acids (all except methionine), 0.14mCi/ml ³⁵S-methionine, 1.2 mM ATP, 120 µM GTP, 18 mM creatine pgosphate, 0.3 mM spermidine, 1.5 mM magnesium acetate, 25 mM potassium acetate, 125 mM KCl, 1.33 mM EGTA, 90 µg/ml calf liver t-RNA, 60 µg/ml creatine kinase, 0.3 µg/9 µl mRNA.

After the reaction, the mixture was treated with a glycosidase to confirm the presence or absence of the carbohydrate chains attached to each of the translation products. Endoglycosidase H (Endo-H (New ENGLAND BioLabs)) was used for the reaction. At first, one tenth volume of the denaturation buffer (10×) enclosed with the enzyme was added to the reaction mixture, and incubated at 50°C for 10 minutes. Next, each of one tenth volumes of 0.5 M sodium phosphate (pH7.5 at 25°C), and 10% NP-40 was added to the denatured cooled samples at room temperatute, and then 1 µl of Endo-H (500, 000 units/ml) was added to the sample to start the reaction. In a control reaction, water was added instead of Endo-H. After the reaction at 37°C for 2 hours, each of the samples was treated with SDS and resolved by SDS-PAGE (12.5% of polyacrylamide gel concentration). Products labeled by the isotope were detected and quantified by BAS 2000 (Fuji).

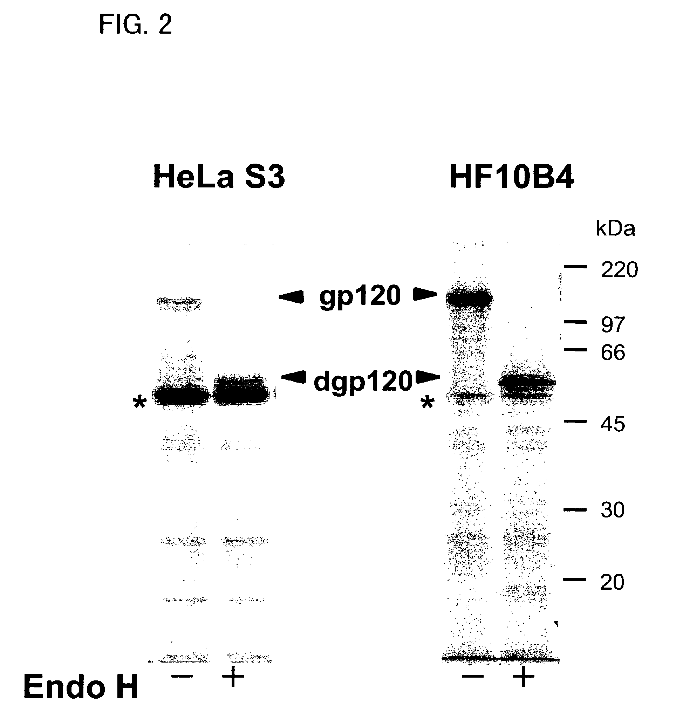

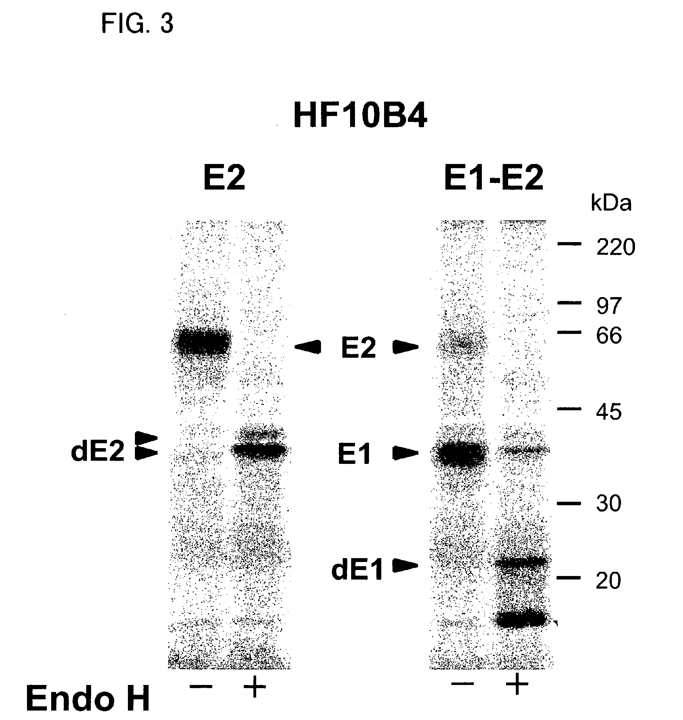

The results were shown in Figs. 2 and 3. Fig.2 shows the results of SDS-PAGE of the samples of the translation reaction of EMCV-HIVgp120-poly-A encoding the envelop protein gp120 of HIV. The reactions were carried out by using the extract of HeLa S3 cells (left panel), and the extract of HF10B4 cells (right panel), and then incubated in the presence or absence of Endo-H. Arrows indicated both of the translation products added with carbohydrate chain (gp120) and those digested with Endo-H (dgp120). Asterisks (*) showed the products without glycosylation. The difference of the detected band densities indicated that, while most of the translation product of HeLa S3 cell extract was not glycosylated, more than 80% of the product of HF10B4 cell extract was glycosylated. It is also recognized that the molecular weight of the products treated with Endo-H (dgp120) was decreased by only a mass of carbohydrate chain.

Fig. 3 shows the result of translation reaction of EMCV-HCV-E2-poly-A and EMCV-HCV-E1-E2-poly-A, using the extract of HF10B4 cells. After the reaction, the mixture was further incubated in the presence or absence of Endo-H in the same way of Fig.2, and then loaded on SDS-PAGE. Arrows showed the translation products added with glycosylation (E1, E2), and digested E1 and E2 by Endo-H (dE1, dE2), in respective reactions. As the results, both products were recognized to be glycosylated. The E1-E2 product was detected as respective proteins of E1 and E2, but not as a fused protein. Therefore, the envelop protein is probably glycosylated through an appropriate post-translational processing.

## Claims

1. A process for producing a protein with a post-translational modification in a cell-free protein synthesis system comprising
preparing a cell extract from cultured cells derived from an immortalized mammalian cell line having an enhanced activity of protein secretion, and
adding an mRNA encoding a glycoprotein to said cell extract.

2. The process of claim 1, wherein said cultured cells are suspension cells.

3. The process of claim 1 or 2, wherein said cultured cells are derived from a hybridoma cell that produces a monoclonal antibody.

4. The process of claim 3, wherein said hybridoma cell is a cell derived from human.

5. The process of any one of claims 1 to 4, wherein said mRNA encoding a glycoprotein is a virus-derived RNA.

6. A composition for use in a synthesis of protein in a cell-free system, comprising a cell extract from cultured cells derived from an immortalized mammalian cell line having an enhanced activity of protein secretion.

7. The composition of claim 6, further comprising an amino acid, a buffer solution, a salt, a nucleotidetriphosphate, and an energy substance.

8. The composition of claim 6 or 7, wherein said cultured cells are derived from a hybridoma cell that produces a monoclonal antibody.

9. The composition of claim 8, wherein said hybridoma cell is a cell derived from human.

10. Use of the composition of any one of claims 6 to 9 for producing a glycoprotein.
